Europäisches Patentamt

European Patent Office

Office européen des brevets

(19)

(11) Publication number: **0 200 548**
**A2**

(12) # EUROPEAN PATENT APPLICATION

(21) Application number: **86303297.5**

(22) Date of filing: **30.04.86**

(51) Int. Cl.⁴: **A 61 K 7/32**

(30) Priority: **02.05.85 US 729518**

(43) Date of publication of application:
**05.11.86 Bulletin 86/45**

(84) Designated Contracting States:
**AT BE CH DE FR GB IT LI LU NL SE**

(71) Applicant: **CALGON CORPORATION**
**Route 60-Campbell's Run Road**
**Robinson Township Pennsylvania 15205(US)**

(72) Inventor: **Klein, William L.**
**66 Edison Avenue**
**Nutley New Jersey 07110(US)**

(72) Inventor: **Sykes, Arthur R.**
**23 Brooktree Road**
**East Windsor, 08520(US)**

(74) Representative: **Crampton, Keith John Allen et al,**
**D YOUNG & CO 10 Staple Inn**
**London WC1V 7RD(GB)**

(54) Deodorant compositions containing cationic polymers.

(57) Stable deodorant compositions comprise at least one antimicrobial agent and a copolymer of dimethyldialkyl ammonium chloride with acrylamide or a polymer of dimethyldialkyl ammonium chloride cationic polymer, which serve to enhance the residual efficacy of the antimicrobial agent on a surface.

EP 0 200 548 A2

# DEODORANT COMPOSITIONS CONTAINING CATIONIC POLYMERS

The invention relates to deodorant compositions comprising at least one antimicrobial agent.

There are numerous references in the literature to cosmetic and personal-use detergent compositions such as shampoos and anti-dandruff rinse conditioners containing anionic and/or cationic polymers, an active agent, surfactants, emollients, and other additives and preservatives commonly used in the industry. While no such references disclose the use of the specific cationic polymers without surfactants, literature references that disclose cosmetic and personal-use detergent formulations containing either or both anionic and cationic polymers are discussed below.

U.S. Patent US-A-3,761,417 discloses detergent compositions containing particle-deposition-enhancing agents, more specifically, detergent and personal-use toilet detergent bars containing water-insoluble particles such as antimicrobial agents, organic surfactants and cationic polymers. Surfactants are an essential ingredient of the compositions. DMDAAC is specifically mentioned as a possible cationic polymer.

U.S. Patent US-A-3,769,398 discloses non-anionic hair shampoo formulations containing an active ingredient (detergent such as betaines, sulphobetaines, amine oxides and mixtures thereof), a water-soluble polymer (polyethyleneimine-ethylene oxide or propylene oxide) and propoxylated polyethylenimines.

U.S. Patent US-A-4,329,335 describes an amphoteric-nonionic anti-dandruff shampoo containing an active agent (1-imidazalyl-1-(4-chlorophenoxy)-3,3-dimethylbutan-2-one), and amphoteric surfactants, polyoxyethylene hexitan mono or polycarboxylic fatty acid esters, tertiary amine oxides, fatty acid mono- or di-ethanolamides and optionally a polymerized quarternized ammonium compound, of which DMDAAC is disclosed as preferred.

European Patent Application EP-A-74,819 discloses an anti-dandruff cream rinse conditioner containing zinc pyrithione, homopolymers of DMDAAC or copolymers of DMDAAC-acrylamide, glucan or guar gum and hydroxyethylcellulose.

European Patent Application EP-A-55,857 teaches preparation of topically applied compositions comprising ultraviolet-light-absorbing materials and film-forming polymers that exhibit enhanced protection from erythema-causing radiation.

In accordance with this invention, stable and effective deodorant compositions comprise at least one antimicrobial agent, a cationic polymer that is a poly-dimethyldialkyl ammonium chloride-acrylamide copolymer or a dimethyldialkyl ammonium chloride polymer, preferably in water. The amount of polymer is sufficient to enhance the deposition and/or retention, and hence the residual efficacy, of the antimicrobial agent on the intended surface for application of the composition. Representative surface areas are the skin and hair of the body.

The formulation should be suitable for topical application and may comprise in addition to an effective amount of the antimicrobial agent and the selected cationic polymer, an anti-perspirant and commonly used cosmetically acceptable excipients such as buffering and/or preservative agents.

The formulation is made by agitating the polymer with de-ionized water, adding an emulsifier with continued agitation, heating to a temperature ranging from $65^{\circ}$ to $90^{\circ}$C (preferably $75^{\circ}$ to $80^{\circ}$) until solution is obtained, and continuing agitation while adding the antimicrobial agents, followed by preservatives, thickening agents, buffers, dyes and/or fragrances, and optionally an antiperspirant agent, and ultimately adjusting the formulation by adding the remainder of the de-ionized water while maintaining the temperature.

The amount of the antimicrobial agent varies over a wide range and depends on the specific agent used. Generally, the antimicrobially effective amount is considerably less than that already known to obtain the desired results. Specifically, the amount of antimicrobial agent used in the formulations of the present invention may be from 0.01% to 5% (preferably 1% to 2%) of the total formulation weight.

The amount of polymer in the compositions of the invention usually ranges from 0.01% to 12% polymer (preferably 1% to 3%) by weight of the formulation. The remaining portion of the total composition contains the antimicrobial agent and conventional acceptable excipients.

Examples of copolymers of dimethyldiallyl ammonium chloride (DMDAAC) and acrylamide, are those sold under the registered trade marks Merquat S and Merquat 550, which are manufactured by Calgon Corporation. The preferred product is Merquat S. Merquat 550 and S copolymers have an intrinsic viscosity of 4.2 ± 0.2 and contain about 50 percent by weight of DMDAAC and 50 percent by weight of acrylamide. Although any ratio of DMDAAC to acrylamide will work, the preferred ratio is 10 to 75 percent, especially 25 to 75 percent, by weight of DMDAAC, balance acrylamide. Suitable homopolymers of DMDAAC have a molecular weight ranging from 20,000 to 2,500,000, preferably from 200,000 to 300,000 and an intrinsic viscosity of 0.1 to 2.5, preferably from 0.7-0.9. The preferred polymers are the copolymers of DMDAAC/AM.

Representative antimicrobial agents used in the practice of the invention are trichlocarban (3,4,4'-trichlorocarbanilide), triclosan (2,4,4'-trichloro-2'-hydroxy-diphenyl ether), benzalkonium chloride, zinc phenolsulphonate and zinc ricinoleate.

Representative anti-perspirants that can be used in the compositions of the invention are aluminium zirconium complex, aluminium chlorohydrate, and aluminium chlorohydrex P.G.

Representative emmollients, humectants and moisturizing agents that can be used in compositions of the invention are $C_{12}$-$C_{15}$ alcohol benzoates, sorbitol, glycerin, propylene glycol (PEG), lanolin, vegetable oils, mineral oils, isopropyl myristate, aloe vera and jojoba oil.

Other cosmetically acceptable excipients that the formulation may contain are thickening agents, buffering agents and preservatives. Suitable water-soluble preservatives are sodium bisulphite, sodium thiosulphate, sodium ascorbate, benzalkonium chloride, glydant chlorobutanol, thimerosal, phenylmercuric borate, Dowicil 200, parabens, 1,2-dibromo-2,4-dicyanobutane, benzyl alcohol and phenylethanol. Suitable thickening agents are that sold under the registered trade mark Cab-O-Sil M5 made by Cabot Corporation, sodium stearate, magnesium aluminium silicate, and hydroxyethyl cellulose. The amounts of agents may be from 0.05 to 50%, preferably 1 to 5%, by weight. Suitable water-soluble buffering agents are alkali metal or alkaline-earth metal carbonates, phosphates, bicarbonates, citrates, borates, acetates, acid anhydrides and succinates, such as sodium phosphate, citrate, borate, acetate, bicarbonate and carbonate. These agents may be present in amounts sufficient to maintain an optimum pH of the system in the range 2 to 9. As such the buffering agent can make up as much as 20% by weight basis of the total composition.

Studies were conducted on the degree of inhibition of antimicrobial activity of deodorants of pig skin with and without a polymer. The organisms tested were mixed inoculum, <u>Staphylococcus</u> <u>aureus</u> and <u>Escherichia</u> <u>coli</u> using a control that contained no polymer (Example 1) and Merquat 100 and Merquat S as polymers are detailed in Example 2 and 3, respectively. The results are shown in Table I below:

## TABLE I

### ANTIMICROBIAL ACTIVITY OF DEODORANTS ON PIG SKIN

|  | Zone of Inhibition Diameter (in mm*) | | | |
|---|---|---|---|---|
| Test Organism | Control (Example 1) | Merquat 100 (Example 2) | Merquat S (Example 3) | No Treatment |
| mixed innocular | 10.0 | 13.3 | 14.6 | 0 |
| Staphylococcus aureus | 27.3 | 30.3 | 35.3 | 0 |
| Escherichia coli | 18.3 | 22.6 | 25 | 0 |

*millimeter

NOTES:

A zone of inhibition of growth of the test organism under and surrounding the discs shows that the compound being tested is microbiologically active.

A zero value indicates microbial growth under the disc and no microbiological activity against that test organism.

*These numbers indicate the average of triplicate results.

As noted from Table I, deodorant samples containing Merquat demonstrate better activity than deodorant without Merquat. The enhanced microbiological activity of the deodorant formulation in the presence of the polymer may be due to improved substantivity, retention on skin surface, or improved diffusion of the deodorant's active ingredient.

6

The following examples illustrate the preparation of various deodorant formulations of the invention. The Examples should be construed as illustrations rather than limitations thereof.

## EXAMPLE 1

### Stick Deodorant Control Formulation*

| NO. | PHASE | INGREDIENT | % BY WEIGHT |
|-----|-------|------------|-------------|
| 1 | A | deionized water | 14.00 |
| 2 | A | propylene glycol | 74.80 |
| 3 | B | Cab-O-Sil M5 | 2.00 |
| 4 | C | triclosan | 0.20 |
| 5 | D | sodium stearate | 8.50 |
| 6 | E | fragrance | 0.50 |
| | | q.s. | 100.00 |

*Without polymer

With rapid agitation, phase A ingredients were throughly mixed with phase B and the mixture heated to 80°C while adding phase C. When dissolved, phases D and E were successively added, agitated until dissolved and q.s. to 100%. The resulting mixture was then poured into the suitable containers for personal care application. (Note: Maintain 80°C temperature while making additions.)

## EXAMPLE 2

### Stick Deodorant

| NO. | PHASE | INGREDIENT | % BY WEIGHT |
|-----|-------|------------|-------------|
| 1 | A | deionized water | 14.00 |
| 2 | A | propylene glycol | 72.80 |
| 3 | B | Cab-O-Sil M5 | 2.00 |
| 4 | C | triclosan | 0.20 |
| 5 | D | sodium stearate | 8.50 |
| 6 | E | fragrance | 0.50 |
| 7 | F | Merquat 100 | 2.00 |
|   |   | q.s. | 100.00 |

The reaction conditions and procedure of Example 1 was followed except that "Merquat 100" was substituted for "Merquat S" to obtain the corresponding product.

## EXAMPLE 3

### Stick Deodorant

| NO. | PHASE | INGREDIENT | % BY WEIGHT |
|-----|-------|------------|-------------|
| 1 | A | deionized water | 14.00 |
| 2 | A | propylene glycol | 71.80 |
| 3 | B | Cab-O-Sil M5 | 2.00 |
| 4 | C | triclosan | 0.20 |
| 5 | D | sodium stearate | 8.50 |
| 6 | E | fragrance | 0.50 |
| 7 | F | Merquat S | 3.00 |
|   |   | q.s. | 100.00 |

With rapid agitation, phase A ingredients, were throughly mixed with phase B and the resulting mixture heated to 80°C while adding phase C. When dissolved, phases D and E were successively added and agitated until dissolved. With continued agitation, phase F was added to the mixture and q.s. to 100%. The resulting mixture was then poured into the suitable containers for personal care application. (Note: Maintain 80°C temperature while making additions).

Following the above reaction conditions and procedure, except that when trichlocarban, benzalkonium chloride, zinc phenosulfonate or benzalkonium chloride is substituted for triclosan, there is obtained the corresponding stick deodorant formulation.

## EXAMPLE 4

### Roll-on Deodorant

| NO. | PHASE | INGREDIENT | % BY WEIGHT |
|---|---|---|---|
| 1 | A | deionized water | 85.60 |
| 2 | A | Merquat 100 | 2.00 |
| 3 | B | ppg-15 stearyl ether | 4.00 |
| 4 | B | steareth-21 | 0.60 |
| 5 | B | steareth-2 | 2.60 |
| 6 | C | triclosan | 1.00 |
| 7 | C | peg 8 | 4.00 |
| 8 | D | preservative | 0.20 |
| | | q.s. | 100.00 |

In separate vessels, the ingredients of phases A and B were heated to 75°C. With agitation, phase B was added to phase A, cooled to 60°C and phase C ingredients were added. Agitation was continued and the reaction mixture was cooled and phase D ingredient added.

## EXAMPLE 5

### Cream Deodorant

| NO. | PHASE | INGREDIENT | % BY WEIGHT |
|-----|-------|-----------|-------------|
| 1 | A | deionized water | 36.00 |
| 2 | A | sorbitol | 3.00 |
| 3 | A | Merquat 100 | 2.00 |
| 4 | B | cetyl alcohol | 4.00 |
| 5 | B | glycerol stearate and peg 100 stearate | 10.00 |
| 6 | C | peg 8 | 4.00 |
| 7 | C | triclosan | 1.00 |
| 8 | D | aluminum zirconium complex, preservative q.s. | 40.00 |
| | | | 100.00 |

In separate vessels, the ingredients of phases A and B were heated to 75°C. With agitation, phase B was added to phase A, cooled to 60°C and phase C ingredients were added. Agitation was continued thereby cooling the reaction mixture and phase D ingredients were added.

## EXAMPLE 6

### Roll-on Deodorant

| NO. | PHASE | INGREDIENT | % BY WEIGHT |
|-----|-------|------------|-------------|
| 1 | A | deionized water | 77.00 |
| 2 | A | Merquat S | 3.00 |
| 3 | B | glyceryl stearate and peg 100 stearate | 10.00 |
| 4 | B | cetyl esters | 5.00 |
| 5 | C | triclosan | 1.00 |
| 6 | C | peg 8 preservative q.s. | 4.00 |
|   |   |   | 100.00 |

In separate vessels, the ingredients of phase A and B were heated to 75°C. With agitation, phase B was added to phase A, cooled to 60°C and phase C ingredients added. Agitation was continued thereby cooling the reaction mixture and phase D ingredients were added.

12

## EXAMPLE 7

### Roll-on Deodorant

| NO. | PHASE | INGREDIENT | % BY WEIGHT |
|-----|-------|-----------|-------------|
| 1 | A | deionized water | 84.60 |
| 2 | A | Merquat 100 | 2.00 |
| 3 | B | peg-15 stearyl ether | 4.00 |
| 4 | B | steareth 21 | 0.60 |
| 5 | B | steareth 2 | 2.60 |
| 6 | C | triclosan | 1.00 |
| 7 | C | peg 8 | 4.00 |
| 8 | D | triclocarbon | 1.00 |
| 9 | E | preservative, fragrance and dye q.s. | 100.00 |

In separate vessels, the ingredients of phase A and phase B were heated to 75°C. With continued agitation phase B was added to phase A. Agitation was maintained and the reaction mixture cooled to 60°C while adding phases D and E successively. The reaction mixture was cooled to 25°C.

0200548

## CLAIMS

1.      A deodorant composition comprising at least one antimicrobial agent and a cationic polymer that is a dimethyldialkyl ammonium chloride/acrylamide copolymer or a polydimethyldialkyl ammonium chloride.

2.      A composition as claimed in Claim 1, additionally comprising water.

3.      A composition as claimed in Claim 1 or 2 in which the antimicrobial agent is trichlocarban (3,4,4'-trichlorocarbanilide), triclosan (2,4,4'-trichloro-2'-hydroxy-diphenyl ether), benzalkonium chloride, zinc phenosulphonate or zinc ricinoleate.

4.      A composition as claimed in any preceding claim in which the copolymer contains 10 to 75 percent by weight of dimethyldialkyl ammonium chloride and 90 to 25 percent by weight of acrylamide.

5.      A composition as claimed in Claim 4 in which the copolymer contains 25 to 75 percent by weight of dimethyldialkyl ammonium chloride and 75 to 25 percent by weight of acrylamide.

6.      A composition as claimed in any preceding claim that further comprises cosmetically acceptable excipients.

7.      A composition as claimed in any preceding claim that further comprises, as an anti-perspirant agent, aluminium zirconium complex, aluminum chlorohydrate or aluminum chlorohydrex P.G.

8.      A composition as claimed in any preceding claim in stick, roll-on or cream formulation form.

9.      A composition as claimed in any preceding claim for use in the treatment of body odour.